# EUROPEAN PATENT APPLICATION

(11) **EP 4 442 239 A1**
(43) Date of publication of application: **09.10.2024**
(21) Application number: 22901118.4
(22) Date of filing: 18.11.2022
(51) Int. Cl.: A61K 8/02, A61Q 1/00

(54) **COSMETIC MATERIAL PRODUCTION METHOD**

(30) Priority: 03.12.2021 JP 2021197165
(71) Applicant: Albion Co., Ltd., Tokyo, 104-0031 (JP)
(72) Inventor: KINOSHITA, Masashi, Kumagaya-shi, Saitama 369-0108 (JP); ITO, Masaru, Kumagaya-shi, Saitama 369-0108 (JP); MIYAMOTO, Takafumi, Kumagaya-shi, Saitama 369-0108 (JP); MARUYAMA, Rengo, Kumagaya-shi, Saitama 369-0108 (JP); KAGAYA, Kei, Kumagaya-shi, Saitama 369-0108 (JP)
(74) Representative: Grünecker Patent- und Rechtsanwälte PartG mbB
(86) International application number: PCT/JP2022/042813
(87) International publication number: WO 2023/100685

(57) **Abstract**

In a method for producing a cosmetic material according to the present invention, a powder-attached rod-like cosmetic material (13) is cut into a plurality of cosmetic material units (14), and the plurality of the cosmetic material units (14) are positioned and arranged at predetermined positions. Consequently, the plurality of the cosmetic material units (14) can be arranged with high precision, and the precision of design and pattern of a cosmetic material (1) formed by the arrangement of the plurality of the cosmetic material units (14) is improved, thereby improving the quality of the cosmetic material (1).

## Description

### TECHNICAL FIELD

The present invention relates to a method for producing a cosmetic material.

### BACKGROUND TECHNOLOGY

As one example of a conventional method for producing a cosmetic material, for example, one has been known which is described in a following patent document 1.

The outline will be explained. In the cosmetic material production method, a solid cosmetic material is formed in a manner that two or more powder-attached rod-like cosmetic materials formed by attaching a powder cosmetic material to the outer peripheral sides of rod-like cosmetic materials are accommodated in a square cylindrical container, and these are pushed out from the container at a predetermined amount and are cut in the direction orthogonal to the long axis of the rod-like cosmetic materials.

### PRIOR ART REFERENCE(S)

### PATENT DOCUMENT(S)

Patent Document 1: Japanese Patent No.5891469

### SUMMARY OF THE INVENTION

### PROBLEM(S) SOLVED BY THE INVENTION

In recent years, in a solid cosmetic material, for example, pattern and design are formed by the arrangement of a plurality of cosmetic material units forming the solid cosmetic material. However, in the conventional cosmetic material production method, when powder-attached rod-like cosmetic materials are accommodated in a container, the positioning of the power-attached rod-like cosmetic materials is not considered at all. Consequently, it is difficult to sufficiently ensure the precision of the pattern and design formed by the arrangement of the plurality of the cosmetic material units, and the quality of a cosmetic material deteriorates. There is therefore room for improvement.

Therefore, the present invention is made in consideration of such a technical problem, and an object of the present invention is to improve a method for producing a cosmetic material which is capable of improving the quality of a cosmetic material by arranging a plurality of cosmetic material units with high precision.

### MEANS FOR SOLVING THE PROBLEM(S)

A method for producing a cosmetic material according to the present invention, in one aspect thereof, includes: forming a plurality of cosmetic material units by finely cutting a rod-like cosmetic material formed by forming a cosmetic material in a rod shape, as a cosmetic material cutting step; and positioning and arranging the plurality of the cosmetic material units at respective predetermined positions, after the cosmetic material cutting step, as a cosmetic material arrangement step.

In this way, according to the present invention, after a rod-like cosmetic material is finely cut into a plurality of cosmetic material units, the plurality of the cosmetic material units are positioned and arranged so as to be positioned at predetermined positions. With this, the plurality of the cosmetic material units can be arranged with high precision, and the precision of pattern and design formed by the arrangement of the plurality of the cosmetic material units is improved, thereby improving the quality of the cosmetic material.

In addition, in the method for producing the cosmetic material according to the present invention, as to the positioning of the plurality of the cosmetic material units in the cosmetic material arrangement step, for example, the positioning and arrangement may be carried out by manual work of an operator while using a jig having partitions, or may be automatically carried out based on an arrangement program recorded in advance by an automatic machine such as a robot.

In addition, as another aspect of the method for producing the cosmetic material, it is desirable that, in the cosmetic material arrangement step, the plurality of the cosmetic material units are positioned and arranged onto a pan via a jig having a partition.

In this way, according to the present invention, in the cosmetic material arrangement step, by positioning and arranging the plurality of the cosmetic material units via the jig having the partition, without relying on an automation carried out by, for example, a robot, the plurality of the cosmetic material units can be mechanically arranged with high precision by manual work of an operator. Consequently, while suppressing an increase in capital investment relating to the cosmetic material production, the positioning and arrangement of the plurality of the cosmetic material units can be realized at a low manufacturing cost.

In addition, in yet another aspect of the method for producing the cosmetic material, it is desirable to further include, before the cosmetic material cutting step, attaching a powder cosmetic material to an outer peripheral surface of the rod-like cosmetic material after the forming of the rod-like cosmetic material, as a powder attachment step, wherein, in the powder attachment step, the powder cosmetic material is attached to the outer peripheral surface of the rod-like cosmetic material by rolling the rod-like cosmetic material on the powder cosmetic material.

In this way, according to the present invention, by rolling the rod-like cosmetic material on the powder cosmetic material, a power-attached rod-like cosmetic material is formed. With this, the powder cosmetic material can be uniformly attached to the outer peripheral side of the rod-like cosmetic material, thereby achieving improvement in quality of the cosmetic material.

In addition, in yet another aspect of the method for producing the cosmetic material, it is desirable that the rod-like cosmetic material is transported in a state of being mounted on a conveyor on which the powder cosmetic material has been spread, so as to be arranged in parallel in a travel direction of the conveyor, and rotates on its axis on the powder cosmetic material, which has been spread on the conveyor, by a movement of the conveyor in a pressed state in which the rod-like cosmetic material has been pressed by applying pressure force thereto from a side facing the conveyor during the transportation, so as to automatically attach the powder cosmetic material to the outer peripheral side of the rod-like cosmetic material.

In this way, according to the present invention, the pressure force is applied to the rod-like cosmetic material from the side facing the conveyor on the conveyor on which the powder cosmetic material has been spread in order to rotate the rod-like cosmetic material on its axis by using the power of the conveyor, such that the powder cosmetic material is automatically attached to the outer peripheral side of the rod-like cosmetic material. In this way, by automatically attaching the powder cosmetic material to the outer peripheral side of the rod-like cosmetic material during the transportation of the rod-like cosmetic material, a powder attachment work for attaching the powder cosmetic material to the outer peripheral side of the rod-like cosmetic material by additionally rolling the rod-like cosmetic material on the powder cosmetic material can be omitted, thereby improving the productivity of the cosmetic material.

In addition, in yet another aspect of the method for producing the cosmetic material, it is desirable that the pressure force is applied by a pressing jig disposed so as to face the conveyor, and the pressing jig is arranged inclined such that a distance between the pressing jig and the conveyor gradually increases toward the traveling direction of the conveyor, so as to come in contact with the rod-like cosmetic material at an end portion of the pressing jig which is located on an opposite side of the traveling direction of the conveyor and not to come in contact with the rod-like cosmetic material at an end portion of the pressing jig which is located on a side in the traveling direction of the conveyor.

In this way, according to the present invention, the pressing jig is arranged inclined so as to come in contact with the rod-like cosmetic material at the end portion of the pressing jig which is located on the opposite side of the traveling direction of the conveyor and not to come in contact with the rod-like cosmetic material at the end portion of the pressing jig which is located on the side in the traveling direction of the conveyor. With this, it is possible to smoothly transport the powder-attached rod-like cosmetic material toward the traveling direction of the conveyor, powder-attached rod-like cosmetic material to which the powder cosmetic material has been attached at the end portion on the opposite side in the traveling direction of the conveyor. Consequently, the decreasing of the powder cosmetic material due to the contact of the powder-attached rod-like cosmetic material with the pressing jig after the attachment of the powder cosmetic material is suppressed, and thereby a powder-attached rod-like cosmetic material to which the powder cosmetic material is sufficiently attached can be obtained.

In addition, in yet another aspect of the method for producing the cosmetic material, it is desirable to further include obtaining a solid cosmetic material which is integrally formed by bringing the cosmetic material units into tight contact with each other by press-molding the cosmetic material units, which have been positioned and arranged, so as to be pressed and solidified, after positioning and arranging the cosmetic material units in the cosmetic material arrangement step, as a press-molding step, wherein, in the press-molding step, after removing the jig, a preliminary molding in which the cosmetic material units positioned and arranged on the pan are relatively shallowly pressed is carried out, and the press-molding for pressing the cosmetic material units after the preliminary molding is carried out so as to be pressed deeper than the preliminary molding.

In this way, according to the present invention, in the press-molding step, before the press-molding, the preliminary molding for relatively shallowly pressing the plurality of the cosmetic material units is carried out. In this way, by carrying out the press-molding in two steps, each of the cosmetic material units is pressed stepwise, and the cosmetic material units come in contact with each other easier, and thereby they can come in tight contact with each other excellently. Consequently, the quality of the cosmetic material can be improved further.

In addition, in yet another aspect of the method for producing the cosmetic material, it is desirable to further include, after the press-molding step, exposing a cross section of the solid cosmetic material by peeling a surface of the solid cosmetic material, as a surface peeling step.

In this way, according to the present invention, after the press-molding step, the surface of the solid cosmetic material is peeled so as to expose the cross section of the solid cosmetic material. With this, pattern and design which appear on the surface of the solid cosmetic material can be clearer, and the quality of the solid cosmetic material can be improved further.

In addition, in yet another aspect of the method for producing the cosmetic material, it is desirable to further include, after the surface peeling step, carrying out finishing-press in which the exposed cross section is pressed to such that the surface of the cross section is arranged, as a finishing press step.

In this way, according to the present invention, after the surface peeling step, the exposed cross section of the solid cosmetic material is pressed by the finishing press such that the surface is arranged. With this, the surface roughness of the solid cosmetic material can be made smoother, and feeling in use of the solid cosmetic material can be improved.

### EFFECT OF THE INVENTION

According to the present invention, after cutting a rod-like cosmetic material into a plurality of cosmetic material units, the cosmetic material units are each positioned and arranged, and thereby each of the cosmetic material units can be arranged with high precision. Consequently, the precision of the pattern and design of the cosmetic material formed by the arrangement of the plurality of the cosmetic material units is improved, and the quality of the cosmetic material can be improved.

### BRIEF DESCRIPTION OF THE DRAWINGS

FIG. 1 is a plane view showing a cosmetic material according to the present invention.
FIG. 2 is a drawing schematically showing first and second steps of a method for producing the cosmetic material according to the present invention, and FIG. 2(a) is a side view and FIG. 2(b) is a plane view.
FIG. 3 is a schematic diagram showing a third step of the cosmetic material production method according to the present invention.
FIG. 4 is a drawing schematically showing a fourth step of the cosmetic material production method according to a first embodiment of the present invention, and
FIG. 4(a) is a plane view and FIG. 4(b) is a sectional view taken along an A-A line of FIG. 4(a).
FIG. 5 is a drawing showing steps from a fifth step of the cosmetic material production method according to the first embodiment of the present invention, and FIG. 5(a) is a plane view and FIG. 5(b) is a sectional view taken along a B-B line of FIG. 5(a).
FIG. 6 is a sectional view showing a configuration of a jig used in the fourth step of the cosmetic material production method according to a second embodiment of the present invention.
FIG. 7 is a drawing showing steps from the fifth step of the cosmetic material production method according to the second embodiment of the present invention, and FIG. 7(a) is a plane view and FIG. 7(b) is a sectional view taken along a C-C line of FIG. 7(a).

### MODE FOR IMPLEMENTING THE INVENTION

In the following, an embodiment of a method for producing a cosmetic material according to the present invention will be explained in detail based on the drawings.

### (Explanation of cosmetic material)

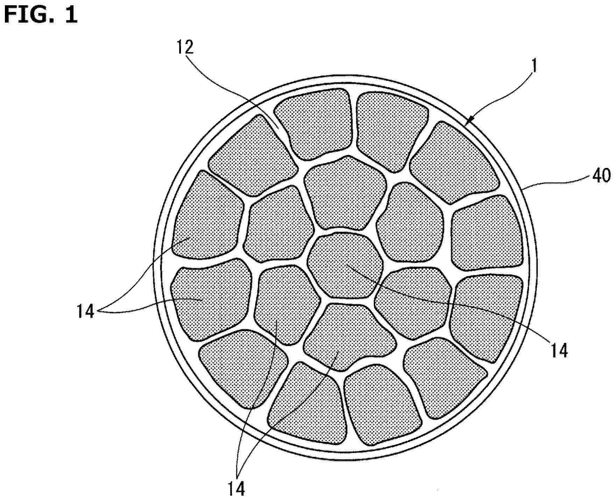

In FIG. 1, there is shown a cosmetic material produced by a method for producing a cosmetic material according to the present invention.

For example, as shown in FIG. 1, a cosmetic material 1 produced by the present invention is one in which a plurality of cosmetic material units 14 having arbitrarily colors are arranged at respective predetermined positions and the plurality of the cosmetic material units 14 are integrated and formed as one solid cosmetic material. In addition, in the cosmetic material 1, the boundary of each of the cosmetic material units 14 is partitioned by a powder cosmetic material 12 having a predetermined color (color different from those of cosmetic material units 14). Then, the cosmetic material 1 is housed in a housing pan 40 made, for example, of metal and is housed in a case (not shown in the drawings) such as a compact case. In addition, materials of the housing pan 40 and the case which is not shown in the drawings can be arbitrarily selected in accordance with specifications of a product.

### [First embodiment]

### (Explanation of method for producing cosmetic material)

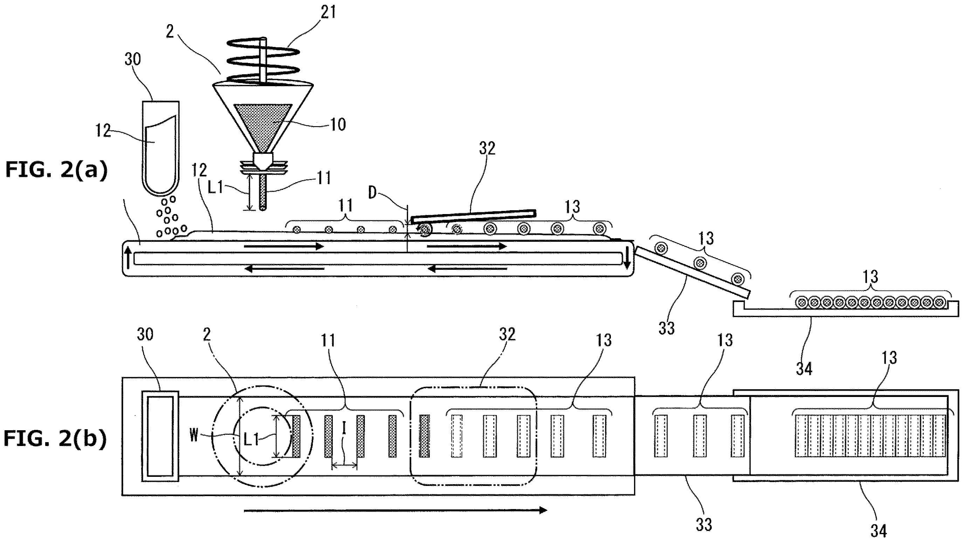

FIG. 2 is a drawing schematically showing first and second steps of a method for producing a cosmetic material according to the present embodiment, and FIG. 2(a) is a side view and FIG. 2(b) is a plane view.

As shown in FIG. 2, in a first step (rod-like cosmetic material molding step) of the cosmetic material production method, by forming a powder cosmetic material having a predetermined color in a rod shape, a rod-like cosmetic material 11 is molded. In addition, as a method for molding the rod-like cosmetic material 11, an arbitrary means such as a press molding and an extrusion molding can be selected in accordance with a cosmetic material to be produced, namely, specifications of a product. In the present embodiment, as an example, a mode for forming the rod-like cosmetic material 11 by the extrusion molding is shown.

That is, in the first step, a rod-like cosmetic material 11 having a substantially cylindrical shape is formed by an extrusion molding machine 2. The extrusion molding machine 2 is a vertical type and is arranged along the vertical direction, and a powder cosmetic material 10 kneaded in the inside thereof by a screw 21 is ejected from the lower end portion of the extrusion molding machine 2, and by cutting the powder cosmetic material 10 ejected therefrom so as to have a predetermined length L1, a rod-like cosmetic material 11 having a substantially cylindrical shape is formed. Here, the extrusion molding machine 2 is arranged on the vertical upper side with respect to a conveyor 31 according to the second step mentioned below so as to face the conveyor 31, and the rod-like cosmetic material 11 formed in the first step can be directly arranged on the conveyor 31 according to the second step and can be continuously transported from the first step to the second step.

In addition, in the first step, it is desirable to provide the extrusion molding machine 2 as many as the kind (number) of colors of the rod-like cosmetic material 11 to be formed in the first step. That is, by providing the extrusion molding machine 2 for each color forming the cosmetic material 1, it is not necessary to change colors in one extrusion molding machine 2, and thereby the productivity of the cosmetic material 1 can be improved.

It is desirable that the rod-like cosmetic material 11 has hardness with stability that can maintain a shape to a certain degree, in consideration of the after-mentioned transporting, rolling and cutting operation. In addition, as to the rod-like cosmetic material 11, an appropriate amount of a volatile solvent can be added as needed. Moreover, the rod-like cosmetic material 11 only needs to have a desired sectional shape in accordance with pattern and design, such as a prism shape in addition to a cylindrical shape illustrated in the present embodiment. In addition, it is desirable that the predetermined length L1 is smaller than a width dimension W of the after-mentioned conveyor 31 and is a length at which a plurality of the after-mentioned cosmetic material units 14 can be obtained without any waste (in a high yield).

Next, in the second step (powder attachment step) of the cosmetic material production method, the rod-like cosmetic material 11 formed in the first step is arranged on the conveyor 31 on which a powder cosmetic material 12 has been spread so as to be parallel in the traveling direction (see, arrow in FIG. 2) of the conveyor 31 at a predetermined interval "I", and by rolling the rod-like cosmetic material 11 on the powder cosmetic material 12 spread on the conveyor 31, the powder cosmetic material 12 is attached to the outer peripheral side of the rod-like cosmetic material 11. In addition, the powder cosmetic material 12 is housed in a powder housing 30 disposed at the end portion on the opposite side of the traveling direction of the conveyor 31 and is supplied from the lower part of the powder housing 30 onto the conveyor 31. Here, in the present embodiment, although a mode in which the powder cosmetic material 12 is attached to the whole circumference on the outer peripheral side of the rod-like cosmetic material 11 is illustrated as an example, the powder cosmetic material 12 is not only attached to the whole outer peripheral side of the rod-like cosmetic material 11, but may also be attached, for example, to part of the outer peripheral side of the rod-like cosmetic material 11.

Specifically, in the second step, a pressing jig 32 is provided in the middle of the conveyor 31 so as to face the upper side of the conveyor 31, with a distance D therebetween in which the rod-like cosmetic material 11 to be transported by the conveyor 31 can come in contact with the pressing jig 32. Here, the pressing jig 32 is disposed in an inclined state such that the distance D with the conveyor 31 gradually increases toward the traveling direction of the conveyor 31. With this, the pressing jig 32 comes in contact with the rod-like cosmetic material 11 at the end portion thereof on the opposite side in the traveling direction of the conveyor 31 and does not come in contact with the rod-like cosmetic material 11 (power-attached rod-like cosmetic material 13) at the end portion thereof on the side in the traveling direction of the conveyor 31.

Then, in the second step, when the rod-like cosmetic material 11 passes under the pressing jig 32 during the transportation by the conveyor 31, the pressure force is applied to the rod-like cosmetic material 11 from the upper side by the pressing jig 32, and the rotation force (torque) is generated to the rod-like cosmetic material 11 from the lower side in the tangential direction (traveling direction of conveyor 31) of the cross section of the rod-like cosmetic material 11 by the movement of the conveyor 31, and thereby the rod-like cosmetic material 11 rotates on its axis on the powder cosmetic material 12 which has been spread on the conveyor 31. By the rotation, the powder cosmetic material 12 is attached to the outer peripheral side of the rod-like cosmetic material 11, and the powder-attached rod-like cosmetic material 13 is formed. The powder-attached rod-like cosmetic material 13 is transported by the conveyor 13 at an interval which is the same as the interval "I", rolls on an inclined plate 33 provided so as to be inclined downward from the conveyor 31, and then is discharged to a discharging tray 34 one after another.

in addition, the attachment work of the powder cosmetic material 12 to the rod-like cosmetic material 11 is not limited to the automatic application by the conveyor 31 and the pressing jig 32, and, for example, the powder cosmetic material 12 can also be applied to the outer peripheral side of the rod-like cosmetic material 11 by manual work by rolling (rotating) the rod-like cosmetic material 11 by manual work on the powder cosmetic material 12 spread on a flat case (tray).

In addition, as to the powder cosmetic material 12, an arbitrary form can be selected such as a powdered form, a granular form, an aggregated form and a slurry form, in accordance with a form of the cosmetic material. As to the attachment work of the powder cosmetic material 12, an arbitrary means can also be adopted in accordance with a form of the powder cosmetic material 12. For example, in case where the powder cosmetic material 12 has a powder form or a granular form, in addition to the rolling of the rod-like cosmetic material 11 on the powder cosmetic material 12, the powder cosmetic material 12 can also be attached to the outer peripheral side of the rod-like cosmetic material 11 by, for example, sprinkling the powder cosmetic material 12 to rod-like cosmetic material 11 during rolling. Alternately, for example, in case where the powder cosmetic material 12 has a slurry form, in addition to the attachment of the powder cosmetic material 12 by rolling the rod-like cosmetic material 11 on the powder cosmetic material 12 having a slurry form, the powder cosmetic material 12 may be attached to the outer peripheral side of the rod-like cosmetic material 11 by, for example, immersing the rod-like cosmetic material 11 into the slurry-form powder cosmetic material 12.

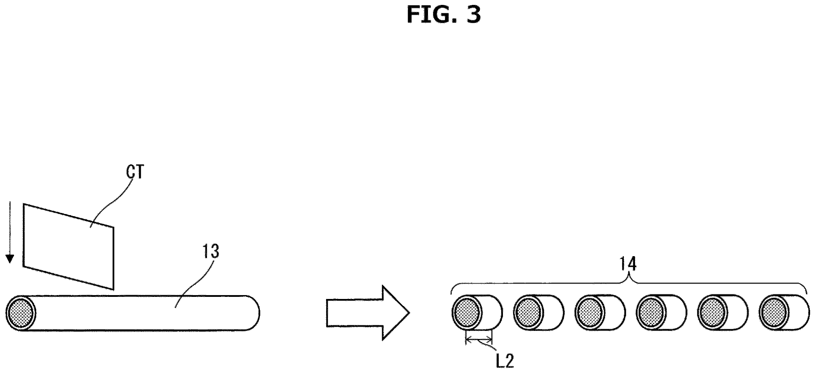

FIG. 3 is a schematic diagram showing a third step of the cosmetic material production method according to the present embodiment and shows perspective views of a powder-attached rod-like cosmetic material 13 and a powder-attached cosmetic material unit 14.

As shown in FIG. 3, in the third step (cosmetic material cutting step) of the cosmetic material production method, the powder-attached rod-like cosmetic material 13 discharged to a discharging tray 34 in the second step is cut by a cutter CT such that powder-attached cosmetic material units 14 each having a predetermined length L2 are formed. The predetermined length L2 is set to be higher than the height of a peripheral wall 402 of the housing pan 40, so as to be the same size as the height of the peripheral wall 402 of the housing pan 40 after the after-mentioned press molding and surface cutting.

In addition, in the present embodiment, as to the third step, although a mode in which the powder-attached rod-like cosmetic material 13 is cut along the direction orthogonal to the long axis direction of the powder-attached rod-like cosmetic material 13 is illustrated as an example, the present invention is not limited to this mode, and the powder-attached rod-like cosmetic material 13 can be cut in an arbitrary direction in accordance with pattern and design of a solid cosmetic material to be produced. In other words, in the third step, the powder-attached rod-like cosmetic material 13 may be cut in the direction crossing the long axis direction of the powder-attached rod-like cosmetic material 13 at a non-right angle or may be cut in parallel to the long axis direction of the powder-attached rod-like cosmetic material 13.

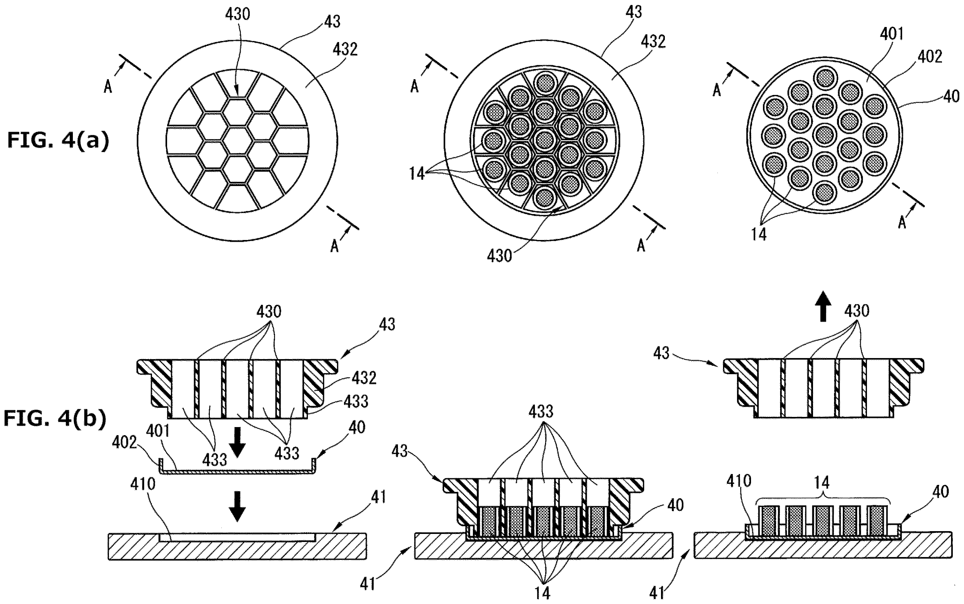

FIG. 4 is a drawing schematically showing a fourth step of the cosmetic material production method according to the first embodiment of the present invention, and FIG. 4(a) is a plane view and FIG. 4(b) is a sectional view taken along an A-A line of FIG. 4(a).

As shown in FIG. 4, in the fourth step (cosmetic material arrangement step) of the cosmetic material production method, first, a housing pan 40 opened toward the vertically upper side is set to a circular recessed portion 410 provided on the upper surface at the middle of a holder member 41 having a substantially thick plate shape, and a jig 43 equipped with partitions 430 is inserted into the housing pan 40 set thereto from the vertically upper side. The jig 43 is formed so as to have a diameter which is stepwisely reduced from one end side toward the other end side and is integrally formed with an insertion portion 431 which is formed on the distal end side so as to have a minimum diameter and is capable of being inserted into the housing pan 40, and a base portion 432 which is formed on the base end side so as to have a large diameter and is seated on the upper end portion of the peripheral wall 402 of the housing pan 40.

Next, the cosmetic material units 14 of each color are inserted into the housing pan 40 via a plurality of insertion holes 433 formed by the partitions 430 of the jig 43, and then are positioned and arranged at predetermined positions. After the completion of the positioning and arrangement of the cosmetic material units 14 of each color, the jig 43 is removed from the housing pan 40. Here, in the present embodiment, in the arrangement work of the cosmetic material units 14 of each color, as an example, a mode is shown in which the cosmetic material units 14 of each color are manually arranged by an operator (worker) by using the jig 43 having the partitions 430.

In other words, in the fourth step, the cosmetic material units 14 of each color can also be automatically arranged by, for example, a robot. That is, by programing the arrangement of the cosmetic material units 14 of each color to be arranged to the housing pan 40 in advance, the cosmetic material units 14 of each color may be positioned and arranged at predetermined positions based on the program. In addition, in case where the positioning and arrangement of the cosmetic material units 14 of each color are automatically carried out by using, for example, a robot, the jig 43 equipped with the partitions 430 for positioning and arranging the cosmetic material units 14 of each color is unnecessary and can be omitted.

In addition, in the fourth step, after positioning and arranging the cosmetic material units 14 of each color, for example, by photographing the arrangement of the cosmetic material units 14 of each color with a camera which is not shown in the drawings, the quality (nondefective/defective) of the arrangement of the cosmetic material units 14 of each color may be determined based on the photographed image. By using the determination means, the precision of the positioning and arrangement of the cosmetic material units 14 of each color can be ensured, and thereby the yield of the cosmetic material 1 can be enhanced.

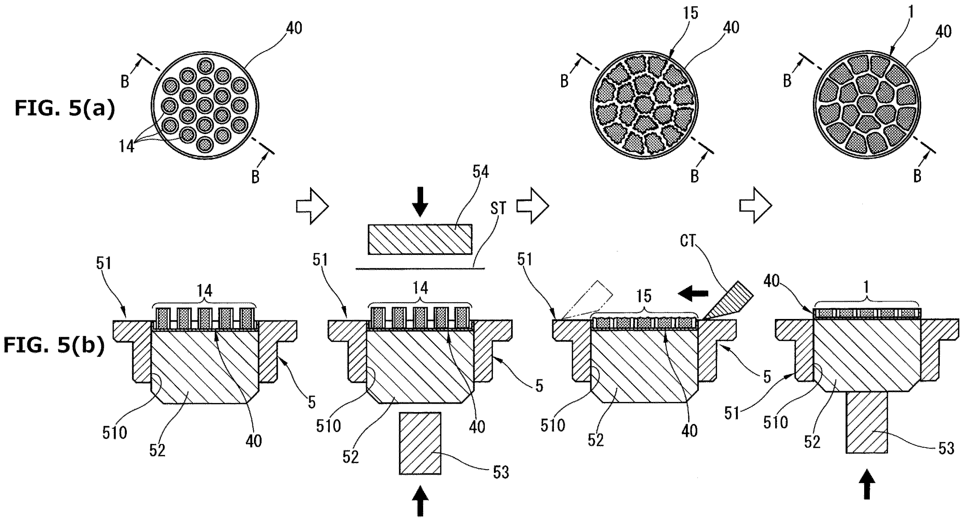

FIG. 5 is a drawing showing an example of steps from a fifth step of the cosmetic material production method according to the first embodiment of the present invention, and FIG. 5(a) is a plane view and FIG. 5(b) is a sectional view taken along a line B-B of FIG. 5(a).

As shown in FIG. 5, in the fifth step (press-molding step) of the cosmetic material production method, first, the housing pane 40 in which the cosmetic material units 14 of each color have been positioned and arranged in the fourth step is set to a press machine 5. The press machine 5 is provided with a substantially cylindrical fixed mold 51 having a mold housing portion 510 formed by penetrating the middle part along the vertical direction, a moving mold 52 slidably housed in the mold housing portion 510 of the fixed mold 51, a ram cylinder 53 disposed on the vertically lower side of the moving mold 52 so as to push the moving mold 52 to the vertically upper side, and a press pad 54 disposed facing the vertically upper side of the moving mold 52 so as to press the upper end surfaces of the cosmetic material units 14 of each color by coming in contact with the upper end surfaces of the cosmetic material units 14 of each color.

Next, when the housing pan 40 is set to the press machine 5, after the press pad 54 moves downward and comes in contact with the upper end surfaces of the cosmetic material units 14, the ram cylinder 53 moves upward and the moving mold 52 is pushed toward the vertically upper side, namely, toward the press pad 54 side. Consequently, the housing pan 40 moves upward together with the moving mold 52, and the whole of the cosmetic material units 14 positioned and arranged in the housing pan 40 is sandwiched between the moving mold 52 and the press pad 54. Then, the cosmetic material units 14 are pressed by the pressing action, are laterally expanded, and push each other in the horizontal direction, and then are brought into tight contact with each other so as to be integrated. With this, a cosmetic material unit assembly 15 in which the cosmetic material units 14 of each color are integrated is formed.

Here, in the fifth step, when the cosmetic material units 14 are pressed by the press pad 54, it is desirable to interpose a paper sheet member ST between the communication material units 14 and the press pad 54. In this way, by interposing the sheet member ST between the cosmetic material units 14 and the press pad 54, a problem that the cosmetic material units 14 adhere to the press pad 54 can be suppressed. Consequently, the upper surface of the cosmetic material unit assembly 15 after the press can be formed smooth.

After the completion of the fifth step, in a sixth step (surface peeling step) of the cosmetic material production method, by moving the cutter CT in the horizontal direction so as to peel the upper end surface of the cosmetic material unit assembly 15 which has been pressed by the pressing action, the upper end portion of the cosmetic material unit assembly 15 is thinly cut. With this, the cross section of the cosmetic material unit assembly 15 is exposed, and a cosmetic material 1 having a smooth surface is formed. After that, an air cylinder 55 moves upward to push the moving mold 52 toward the vertically upper side, and the cosmetic material 1 is discharged together with the housing pan 40, and the production of the cosmetic material 1 is completed.

### (Working effect of the present embodiment)

In the conventional cosmetic material production method, when powder-attached rod-like cosmetic materials are housed in a container, the positioning of the powder-attached rod-like cosmetic materials is not considered at all. Consequently, it is difficult to sufficiently ensure precision of the pattern and design formed by the arrangement of the cosmetic material units, and the quality of a cosmetic material deteriorates. There is therefore room for improvement.

In contrast to this, according to the cosmetic material production method according to the present embodiment, the following effects can be obtained, and the problem of the conventional cosmetic material production method can be solved.

That is, according to the cosmetic material production method according to the present embodiment, after cutting a powder-attached rod-like cosmetic material 13 into a plurality of cosmetic material units 14, the cosmetic material units 14 are positioned and arranged at predetermined positions. Consequently, the plurality of the cosmetic material units 14 can be arranged with high precision, and the precision of the pattern and design of a cosmetic material 1 formed by the arrangement of the cosmetic material units 14 is improved, thereby improving the quality of the cosmetic material 1.

In addition, in the conventional cosmetic material production method, since powder-attached rod-like cosmetic materials housed in a container are pushed out from the end portion of the container at a predetermined amount and then are cut, it is difficult to dispose a jig for positioning the powder-attached rod-like cosmetic materials in the container. In other words, if a jig for positioning the powder-attached rod-like cosmetic materials was disposed in a container, when the powder-attached rod-like cosmetic materials were pushed out form the end portion of the container, the jig would be pushed out together with the powder-attached rod-like cosmetic materials, and the cutting of the powder-attached rod-like cosmetic materials would be obstructed.

In contrast to this, in the present embodiment, in the fourth step, the plurality of the powder-attached cosmetic material units 14 are positioned and arranged via the jig 43 having the partitions 430. With this, without relying on an automation carried out by, for example, a robot (not shown in the drawings), the plurality of the powder-attached cosmetic material units 14 can be mechanically arranged with high precision by manual work of an operator. Consequently, while suppressing an increase in capital investment relating to the production of the cosmetic material 1, the positioning and arrangement of the plurality of the powder-attached cosmetic material units 14 can be realized at a low manufacturing cost.

In addition, in the present embodiment, in the second step, by rolling the rod-like cosmetic material 11 on the powder cosmetic material 12, the powder cosmetic material 12 can be attached to the outer peripheral side of the rod-like cosmetic material 11. Consequently, the powder cosmetic material 12 can be uniformly attached to the outer peripheral side of the rod-like cosmetic material 11, thereby achieving improvement in quality of the cosmetic material 1.

In addition, in the present embodiment, in the second step, by applying pressure force to the rod-like cosmetic material 11 from the side facing the conveyor 31 on the conveyor 31 on which the powder cosmetic material 12 has been spread, the rod-like cosmetic material 11 is rotated on its axis by using the power of the conveyor 31, and the powder cosmetic material 12 is automatically attached to the outer peripheral side of the rod-like cosmetic material 11. In this way, by automatically attaching the powder cosmetic material 12 to the outer peripheral side of the rod-like cosmetic material 11 during the transportation of the rod-like cosmetic material 11, a powder attachment work for attaching the powder cosmetic material 12 to the outer peripheral side of the rod-like cosmetic material 11 by additionally rolling the rod-like cosmetic material 11 on the powder cosmetic material 12 can be omitted, thereby improving the productivity of the cosmetic material 1.

In addition, in the present embodiment, in the second step, the pressing jig 32 is arranged inclined so as to come in contact with the rod-like cosmetic material 11 at the end portion thereof which located on the opposite side of the traveling direction of the conveyor 31 and not to come in contact with the rod-like cosmetic material 11 (powder-attached rod-like cosmetic material 13) at the end portion thereof which is located on the side in the traveling direction of the conveyor 31. With this, the powder-attached rod-like cosmetic material 13 to which the powder cosmetic material 12 has been attached at the end portion on the opposite side of the traveling direction of the conveyor 13 can be smoothly transported in the traveling direction of the conveyor 31. Consequently, the decreasing of the powder cosmetic material 12 due to the contact of the powder-attached rod-like cosmetic material 13 with the pressing jig 32 after the attachment of the powder cosmetic material 12 is suppressed, and thereby a powder-attached rod-like cosmetic material 13 to which the powder cosmetic material 12 has been sufficiently attached can be obtained.

In addition, in the present embodiment, after the fifth step, the surface of the cosmetic material unit assembly 15 formed in the fourth step is peeled so as to expose the cross section of the cosmetic material unit assembly 15, and the cosmetic material 1 is formed. With this, pattern and design which appear on the surface of the cosmetic material 1 can be made clearer, and the quality of the cosmetic material 1 can be improved further.

### [Second embodiment]

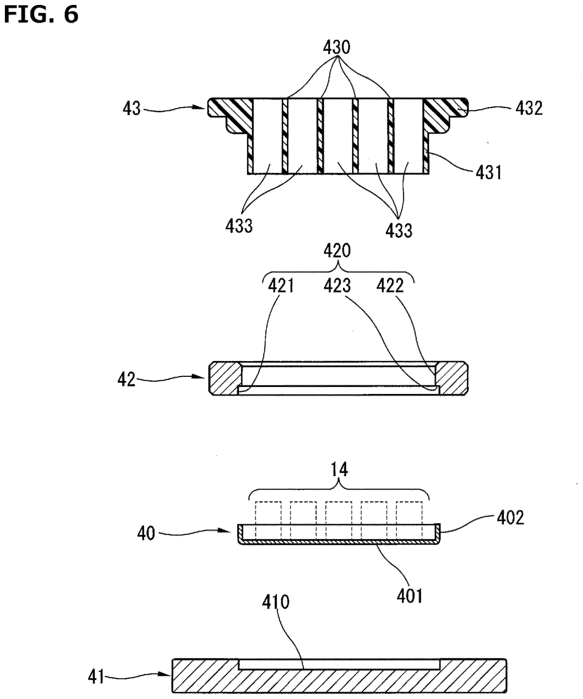

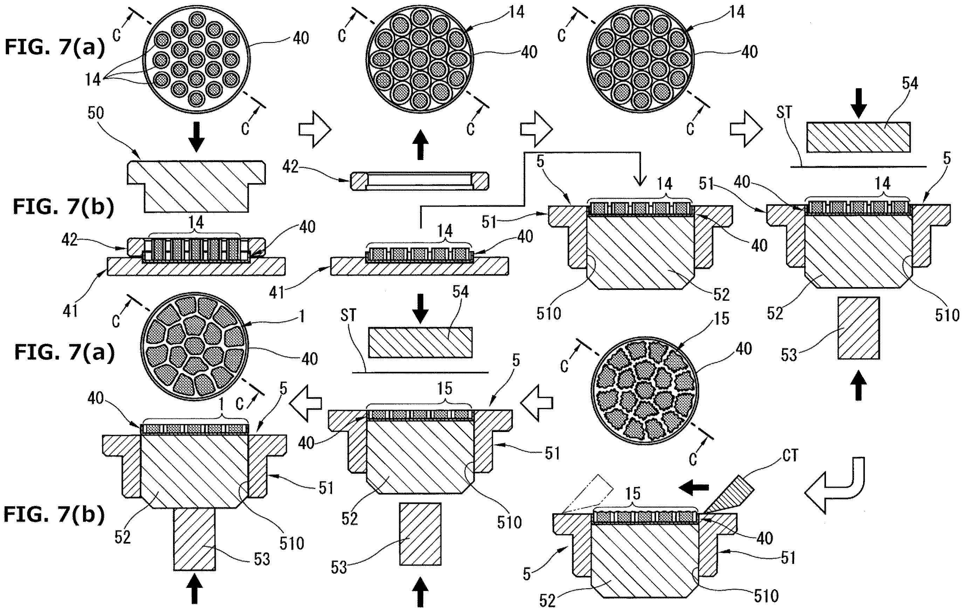

FIGS. 6 and 7 show a second embodiment of the cosmetic material production method according to the present invention, and, in the present embodiment, the steps from the fifth step in the first embodiment are changed. In addition, a basic configuration other than the changes is the same as that of the first embodiment. Therefore, in the same components as those of the first embodiment, the same symbols are applied thereto, and redundant explanation is omitted.

FIG. 6 is a sectional view showing a configuration of a jig used in the fifth step of the cosmetic material production method according to the present embodiment, In addition, FIG. 7 is a drawing showing steps from the fifth step of the cosmetic material production method according to the present embodiment, and FIG. 7(a) is a plane view and FIG. 7(b) is a sectional view taken along a C-C line of FIG. 7(a).

As shown in FIG. 6, in the present embodiment, in the fourth step of the cosmetic material production method, a housing pan 40 is set to a recessed portion 410 formed on the upper surface in the middle of a holder member 41, and a side ring 42 having a substantially annular shape and formed with a through hole 420 capable of receiving the housing pan 40 inside thereof is disposed on the outer peripheral side of the set housing pan 40. After that, a jig 43 equipped with partitions 430 is inserted from the vertically upper side through the through hole 420 of the side ring 42, and cosmetic material units 14 of each color are positioned and arranged to the housing pan 40 via a plurality of insertion holes 433 formed by the partitions 430 of the jig 43. Then, after the positioning and arrangement of the cosmetic material units 14 of each color are completed, the jig 43 is removed from the housing pan 40.

Here, the through hole 420 of the side ring 42 is formed in a step shape and includes a large diameter portion 421 provided at the end portion facing the housing pan 40 so as to hold the outer peripheral side of the peripheral wall 402 of the housing pan 40, a small diameter portion 422 formed in a step shape so as to have a diameter smaller than that of the large diameter portion 421 and formed so as to extend the inner diameter of the peripheral wall 402 of the housing pan 40, and a step portion 423 formed between the large diameter portion 421 and the small diameter portion 422 and capable of being seated on the upper end portion of the peripheral wall 402 of the housing pan 40.

In addition, the jig 43 is formed in a step shape such the diameter decrease from one end side toward the other end side and is integrally formed with an insertion portion 431 formed in a minimum diameter shape on the distal end side and capable of being inserted into the inner side of the housing pan 40, and a base portion 432 formed in a large diameter shape on the base end side so as to be seated on the upper end surface of the side ring 42.

Next, as shown in FIG. 7, in the present embodiment, in the fifth step, a preliminary press pad 44 moves downward toward the housing pan 40 disposed in the recessed portion 410 of the holder member 41 and held by the side ring 42, the distal end portion of the preliminary press pad 44 is inserted into the through hole 420 of the side ring 42, and then the cosmetic material units 14 of each color which have been positioned and arranged in the housing pan 40 are relatively shallowly pressed as a so-called preliminary press. Specifically, the cosmetic material units 14 of each color after the positioning and arrangement are pressed to a degree at which they can come in contact with each other, and then the preliminary press is completed. After the preliminary press is completed, the side ring 42 is removed, and the housing pan 40 is separated from the holder member 41 and is set to the press machine 5. Since the configuration of the press machine 5 is the same as that of the first embodiment, and the detailed explanation is omitted.

Next, when the housing pan 40 is set to the press machine 5, after the press pad 54 moves downward and comes in contact with the upper end surface of each of the cosmetic material units 14, the ram cylinder 53 moves upward, and the moving mold 52 is pushed toward the press pad 54 side. With this, the whole of the cosmetic material units 14 to which the preliminary press has been carried out is sandwiched between the press pad 54 and the moving mold 52, and by being pressed with the press action, each of the cosmetic material units 14 is laterally expanded further, and the cosmetic material units 14 come in contact with each other so as to be integrated. Consequently, a cosmetic material unit assembly 15 formed by integrating the cosmetic material units 14 with each color is formed. In addition, in the fifth step, similar to the first embodiment, it is desirable to interpose a paper sheet member ST between the cosmetic material units 14 and the press pad 54.

Next, after the completion of the fifth step, in a sixth step of the cosmetic material production method, the upper end portion of the cosmetic material unit assembly 15 formed in the fifth step is thinly cut by the cutter CT. With this, the cross section of the cosmetic material unit assembly 15 is exposed. Next, after the completion of the sixth step, in a seventh step (finishing press step) of the cosmetic material production method, the cross section of the cosmetic material unit assembly 15 exposed in the sixth step is pressed slightly by the press pad 54, and the cross section of the cosmetic material unit assembly 15 is press-finished. With this, a cosmetic material 1 having a surface smoother than the cross section exposed by the cutter CT is formed. After that, an air cylinder 55 moves upward so as to push the moving mold 52 toward the vertically upper side, the cosmetic material 1 is discharged together with the housing pan 40, and the production of the cosmetic material 1 is completed.

As the above, in the present embodiment, in the fifth step, before the press molding is carried out, a preliminary molding in which a plurality of cosmetic material units 14 are relatively shallowly pressed is carried out. In this way, by press-molding the plurality of the cosmetic material units 14 in two steps, since each of the cosmetic material units 14 is stepwisely pressed, the cosmetic material units 14 come in contact with each other easier and can be brought into tight contact with each other excellently. Consequently, the quality of the cosmetic material 1 can be improved further.

In addition, in the present embodiment, after the sixth step, the cross section of the cosmetic material unit assembly 15 which has been exposed is press-finished such that the surface is arranged. Consequently, the surface roughness of the cosmetic material 1 can be made smooth further, and thereby feeling in use of the cosmetic material 1 can be improved.

The present invention is not limited to the configuration and mode illustrated in each of the embodiments as an example, and if the present invention has a mode in which the working effects of the present invention can be obtained, various changes might be freely made in accordance with an object to be applied of a mode, costs and the like.

in particular, the color of the cosmetic material units 14 forming the cosmetic material 1 is not limited to one color illustrated in each of the embodiments as an example, and plurality of colors such as two or more colors may be used, and it can be arbitrarily changed in accordance with pattern and design formed to the cosmetic material 1.

In addition, as to the attachment work of the powder cosmetic material 12 carried out in the second step, it is not limited to the mode in which the powder cosmetic material 12 is attached to the outer peripheral side of the rod-like cosmetic material 11 as illustrated in each of the embodiments as an example, and, for example, the cutting step (third step) of the rod-like cosmetic material 11 is carried out in advance, and after the rod-like cosmetic material 11 is cut, the powder cosmetic material 12 may be attached to the outer peripheral side of each of the cosmetic material units 14 obtained by the cutting.

In addition, as to a means for positioning and arranging the plurality of the powder-attached cosmetic material units 14 in the fourth step, the jig 43 having the partitions 430 which is illustrated in each of the embodiments as an example may be used, or it may be automated by using, for example, a robot.

### EXPLANATION OF REFERENCE SIGNS

1... Cosmetic material
11...Rod-like cosmetic material
12...Powder cosmetic material
13... Powder-attached rod-like cosmetic material
14...Cosmetic material unit
15...Cosmetic material unit assembly
2...Extrusion molding machine
21...Screw
30...Powder housing
31...Conveyor
32...Pressing jig
33...Inclined plate
34...Discharging tray
41 ... Holder member
42... Side ring
43...Jig
430...Partition
5...Press machine
51...Fixed mold
52...Moving mold
53...Ram cylinder
54...Press pad
55...Air cylinder
CT...Cutter

## Claims

1. A method for producing a cosmetic material, comprising:
forming a plurality of cosmetic material units by finely cutting a rod-like cosmetic material formed by forming a cosmetic material in a rod shape, as a cosmetic material cutting step; and
positioning and arranging the plurality of the cosmetic material units at respective predetermined positions, after the cosmetic material cutting step, as a cosmetic material arrangement step.

2. The method for producing the cosmetic material according to claim 1, wherein, in the cosmetic material arrangement step, the plurality of the cosmetic material units are positioned and arranged onto a pan via a jig having a partition.

3. The method for producing the cosmetic material according to claim 1, further comprising, before the cosmetic material cutting step, attaching a powder cosmetic material to an outer peripheral surface of the rod-like cosmetic material after the forming of the rod-like cosmetic material, as a powder attachment step,
wherein, in the powder attachment step, the powder cosmetic material is attached to the outer peripheral surface of the rod-like cosmetic material by rolling the rod-like cosmetic material on the powder cosmetic material.

4. The method for producing the cosmetic material according to claim 3, wherein the rod-like cosmetic material is transported in a state of being mounted on a conveyor on which the powder cosmetic material has been spread, so as to be arranged in parallel in a travel direction of the conveyor, and rotates on its axis on the powder cosmetic material, which has been spread on the conveyor, by a movement of the conveyor in a pressed state in which the rod-like cosmetic material has been pressed by applying pressure force thereto from a side facing the conveyor during the transportation, so as to automatically attach the powder cosmetic material to the outer peripheral side of the rod-like cosmetic material.

5. The method for producing the cosmetic material according to claim 4, wherein the pressure force is applied by a pressing jig disposed so as to face the conveyor, and
wherein the pressing jig is arranged inclined such that a distance between the pressing jig and the conveyor gradually increases toward the traveling direction of the conveyor, so as to come in contact with the rod-like cosmetic material at an end portion of the pressing jig which is located on an opposite side of the traveling direction of the conveyor and not to come in contact with the rod-like cosmetic material at an end portion of the pressing jig which is located on a side in the traveling direction of the conveyor.

6. The method for producing the cosmetic material according to claim 2, further comprising obtaining a solid cosmetic material which is integrally formed by bringing the cosmetic material units into tight contact with each other by press-molding the cosmetic material units, which have been positioned and arranged, so as to be pressed and solidified, after positioning and arranging the cosmetic material units in the cosmetic material arrangement step, as a press-molding step,
wherein, in the press-molding step, after removing the jig, a preliminary molding in which the cosmetic material units positioned and arranged on the pan are relatively shallowly pressed is carried out, and the press-molding for pressing the cosmetic material units after the preliminary molding is carried out so as to be pressed deeper than the preliminary molding.

7. The method for producing the cosmetic material according to any one of claims 1 to 6, further comprising, after the press-molding step, exposing a cross section of the solid cosmetic material by peeling a surface of the solid cosmetic material, as a surface peeling step.

8. The method for producing the cosmetic material according to claim 7, further comprising, after the surface peeling step, carrying out finishing-press in which the exposed cross section is pressed such that the surface of the cross section is arranged, as a finishing press step.
